# DEMANDE DE BREVET EUROPEEN

(11) **EP 4 834 332 A1**
(43) Date de publication de la demande: **04.11.2026**
(21) Numéro de dépôt: 26174884.2
(22) Date de dépôt: 27.04.2026
(51) Int. Cl.: A61K 9/20

(54) **COMPOSITION PHARMACEUTIQUE SOLIDE COMPRENANT UNE ARGILE POUR LE TRAITEMENT DE DÉSORDRES GASTRO-INTESTINAUX**

(30) Priorité: 28.04.2025 FR 2504493
(71) Demandeur: Mayoly Pharma France, 92500 Rueil-Malmaison (FR)
(72) Inventeur: Bordier, Juliette, 28100 DREUX (FR); Laigneau, Romy, 28100 DREUX (FR); Martin, Camille, 28100 DREUX (FR)
(74) Mandataire: Gevers & Orès

(57) **Abrégé**

La présente invention se rapporte à une composition pharmaceutique solide, notamment un comprimé, comprenant une argile, notamment une smectite.

## Description

L'invention concerne une composition pharmaceutique solide, notamment un comprimé, comprenant une argile, notamment une smectite.

Les smectites sont un groupe de minéraux argileux classiquement utilisés pour la préparation de médicaments destinés au traitement de désordres gastro-intestinaux tels que les troubles fonctionnels du tractus gastro-intestinal comme la diarrhée, éventuellement chronique ou aigue. D'autres argiles sont utilisées à des fins thérapeutiques comme l'attapulgite.

Actuellement, plusieurs médicaments comprenant de la smectite sont commercialisés pour le traitement des maladies et des troubles du tractus gastro-intestinal d'étiologie différente. On peut citer parmi eux le Smecta^{®}.

L'efficacité et la popularité de ces médicaments tiennent aux propriétés de la smectite qui est un adsorbant d'origine naturelle, qui ne détruit pas la flore digestive et n'affecte pas la motilité intestinale, il n'est pas absorbé par le tractus gastro-intestinal et est excrété sous forme inchangée ; la smectite agit comme pansement formant une barrière protectrice des muqueuses digestives et n'induit pas d'effet secondaire indésirable, elle n'a ainsi quasi aucune contre-indication. Il n'y a pas de restriction d'âge pour recevoir la smectite qui peut ainsi être administrée à des patients de tout âge y compris les enfants en bas âge, dès 2 ans.

La plupart des médicaments à base de smectite actuellement sur le marché sont, à l'instar du Smecta^{®}, formulés sous forme de poudre ou de suspension prête à l'emploi conditionnée dans des sachets dosés à 3 g de diosmectite ; l'administration de ces médicaments se fait par voie orale après mise en suspension de la poudre d'argile dans de l'eau ou directement pour le stick prêt à l'emploi. La prise de ce médicament est cependant parfois mal acceptée pour des raisons organoleptiques (mauvais gout et sensation pâteuse en bouche).

Une solution pour surmonter cet inconvénient serait la mise au point de compositions de smectite sous forme solide à avaler, qui permettraient un mode d'administration par voie orale alternatif aux suspensions buvables.

La mise au point d'une telle formulation solide présente cependant plusieurs difficultés.

Tout d'abord, une première difficulté est l'obtention d'une forme solide qui permette l'administration de la dose thérapeutique de smectite (3 g) tout en ayant un volume suffisamment petit pour être avalé ; dans ce contexte, il est apparu que la formulation d'une poudre de smectite dans des gélules n'est pas satisfaisante car, même en utilisant des tailles de gélules importantes de l'ordre de 1 ml, il faudrait 6 gélules pour administrer une dose de 3 g de smectite, ceci est difficilement acceptable pour des patients, ni réaliste en ce qu'une telle forme nécessiterait jusqu'à 36 gélules à administrer par jour.

La solution visant à comprimer la poudre d'argile avec une densification préalable a donc été retenue, mais celle-ci a soulevé le problème d'obtenir des comprimés avec un aspect visuel satisfaisant (lisse, non friable), une bonne résistance mécanique et stabilité au stockage (comprimés qui ne se fracturent ou s'effritent pas) et surtout avec des propriétés identiques à celles de la formulation en poudre (dosage, distribution granulométrique des particules après dispersion, pouvoir absorbant). D'autant que la smectite est une poudre très fine qui présente des propriétés technologiques qui rendent difficile la préparation sous forme de comprimés : forte abrasivité, faible fluidité et compressibilité, pratiquement insoluble dans l'eau et l'alcool.

Enfin, en raison de la posologie qui requiert une administration de 3 g d'argile, la réalisation de comprimés ayant une taille acceptable nécessite l'utilisation la plus réduite possible d'excipients ; limiter l'utilisation de ces auxiliaires de formulation complexifie encore la mise au point de comprimé de bonne qualité.

Il existe des formulations solides d'argile comme les comprimés Simcere^{®} ou Dimexanol^{®}, qui sont mis en suspension dans de l'eau avant administration, ou encore les comprimés Diatabs^{®} pour chiens qui sont à croquer ; ceux-ci ne sont cependant pas limités en taille puisqu'ils ne sont pas avalés tels quels mais ces formulations ne solutionnent pas le problème de gout et de texture désagréables lors de l'administration.

C'est donc de façon surprenante que la Demanderesse est parvenue à mettre au point des comprimés à haute teneur en argile présentant toutes les qualités requises pour se substituer aux formulations en poudre existantes.

Plus précisément, l'invention se rapporte à des comprimés d'argile consistant en:
- au moins 80% en poids d'argile ;
- de 0,5 à 1,5% en poids de fumarate de stéaryle de sodium;
- de 5 à 15% en poids, de préférence, entre 7 et 10% en poids, encore préférentiellement, entre 8 et 10% en poids, d'hydroxypropylcellulose à titre de liant ;
- optionnellement, de 5 à 15% en poids d'un diluant choisi parmi la cellulose microcristalline, l'amidon et l'amidon prégélatinisé, la maltodextrine, le mannitol, le phosphate dicalcique ; et
- optionnellement, de 2 à 10% en poids d'un désintégrant choisi parmi l'hydroxypropylcellulose faiblement substitué (L-HPC), le croscarmellose sodium, le glycolate d'amidon sodique, la crospovidone, les polysaccharides de soja, l'amidon et l'amidon prégélatinisé.

L'argile peut être choisie parmi la smectite et l'attapulgite ; de préférence, il s'agit de smectite. La teneur en argile dans les comprimés est de préférence d'au moins 83% en poids, encore préférentiellement, comprise entre 85 et 95% en poids.

En particulier, la teneur en argile par comprimé peut être comprise entre 0,4 et 2,4g, et de préférence entre 0,8 et 1,9g. selon un mode de réalisation particulier, les comprimés comprennent 1,5g d'argile.

Le fumarate de stéaryle de sodium joue le rôle de lubrifiant, il est de préférence présent à une teneur comprise entre 0,8 et 1,2% en poids.

Selon un mode de réalisation préféré, l'hydroxypropylcellulose est ultrafine, c'est-à-dire ayant une distribution granulométrique telle que son D₅₀ (taille maximale de 50% des particules) est de 15 à 90 µm et son D₉₀ (taille maximale de 50% des particules) de 35 à 300 µm.

Selon un mode de réalisation particulier, les comprimés selon l'invention ne comprennent pas de désintégrant.

Selon un autre mode de réalisation particulier, les comprimés selon l'invention ne comprennent pas de diluant.

Selon un encore autre mode de réalisation particulier, les comprimés selon l'invention ne comprennent ni désintégrant, ni diluant.

Il n'y a pas de limitation concernant le poids des comprimés selon l'invention ; toutefois, pour l'application envisagée, on préfèrera des comprimés ayant un poids compris entre 0,5 et 2,5 g, de préférence, entre 1 et 2 g.

Les comprimés peuvent avoir toute forme habituellement utilisée pour la fabrication de comprimé à avaler ; à titre d'exemple et sans aucun caractère limitatif, ils peuvent avoir une forme oblongue, comme une ellipse.

Selon un mode de réalisation particulier de l'invention, les comprimés sont sécables, c'est-à-dire qu'ils comportent une géométrie présentant des lignes de fracture permettant de couper facilement le comprimé en plusieurs morceaux ; de préférence, en deux morceaux.

De façon inattendue, bien que contenant une teneur très réduite en excipients (moins de 20% en poids), les comprimés selon l'invention présentent toutes les propriétés requises pour induire les effets thérapeutiques de l'argile, comme cela est objectivé dans la partie expérimentale :
- conformité de la dose en principe actif : la dose en argile dans le comprimé fini doit être comprise entre 95 et 105% de la teneur cible ;
- pouvoir absorbant : cette propriété testée sur l'hémisulfate de strychnine ou l'hexacolbalt (trichlorure de cobaltihexammine) doit conduire à une absorption comprise entre 307 et 575 mg d'hémisulfate de strychnine par g d'argile ou entre 80 et 150 mEq d'hexacolbalt par 100 g d'argile ;
- distribution granulométrique des particules après dispersion : D₉₀ inférieure à 200 µm ;
- temps de désagrégation : il doit être inférieur ou égal à 30 minutes (1800 secondes), et, de préférence, à 15 minutes (900 secondes) ; ce temps de désagrégation est évalué selon la Pharmacopée Européenne 2.9.1 ;
- stabilité mécanique : le test de friabilité doit conduire à une perte en poids des comprimés inférieure à 1% selon la Pharmacopée Européenne 2.9.7.

Ainsi, les comprimés d'argile selon l'invention sont caractérisés par :
- un temps de désagrégation inférieur ou égal à 30 minutes (1800 secondes), de préférence inférieur ou égal à 15 minutes (900 secondes) ; et/ou
- une dose d'argile dans le comprimé fini comprise entre 95 et 105% de la teneur cible ; et/ou
- un pouvoir absorbant tel que l'absorption d'hémisulfate de strychnine est comprise entre 307 et 575 mg d'hémisulfate de strychnine par g d'argile ou l'absorption d'hexacolbalt est comprise entre 80 et 150 mEq d'hexacolbalt par 100 g d'argile ; et/ou
- une distribution granulométrique des particules après dispersion telle que la D₉₀ est inférieure à 200 µm ; et/ou
- une stabilité mécanique telle que la perte en poids des comprimés est inférieure à 1% lors de l'évaluation par le test de friabilité selon la Pharmacopée Européenne 2.9.7..

La présente invention se rapporte encore aux comprimés selon l'invention pour leur utilisation pour le traitement de désordres gastro-intestinaux tels que les troubles fonctionnels du tractus gastro-intestinal comme la diarrhée, éventuellement chronique ou aigue.

En particulier, ils permettent le traitement symptomatique de la diarrhée aiguë chez l'enfant de plus de 2 ans ; le traitement symptomatique de la diarrhée chronique ; le traitement symptomatique des douleurs liées aux affections œsogastroduodénales (dyspepsie fonctionnelle, brûlures d'estomac fonctionnelles) et du reflux gastro-œsophagien et des coliques ; le traitement symptomatique des colopathies fonctionnelles (c'est-à-dire ballonnements et douleurs abdominales, syndrome du côlon irritable) chez l'enfant et l'adulte ; ou encore les douleurs de l'œsophage, de l'estomac, du duodénum ou du côlon.

La présente invention concerne également un procédé de préparation des comprimés selon l'invention.

Selon un premier mode de réalisation, la préparation des comprimés selon l'invention est réalisée par compactage (granulation sèche) de l'argile et comprend les étapes suivantes :
- compactage de l'argile seule ou avec un ou plusieurs excipients ; cette étape peut être réalisée à l'aide d'un compacteur à rouleaux ; la densification de la poudre d'argile est essentielle pour la préparation de comprimés ayant les propriétés souhaitées ;
- mélange du grain obtenu avec le reste des excipients ;
- ajout du fumarate de stéaryle de sodium et mélange ;
- compression et récupération des comprimés.

La mise en œuvre de ces étapes est bien connue de l'homme du métier.

Selon un autre mode de réalisation, la préparation des comprimés selon l'invention est réalisée par un procédé comprenant une granulation humide ; ce procédé comprend les étapes suivantes :
- mélange à sec de l'argile seul ou avec la totalité ou une partie du liant (par exemple, de 3 à 5% de liant en poids du poids total du comprimé) ;
- granulation humide avec la totalité ou une partie du liant s'il n'a pas été ajouté lors de l'étape de mélange à sec ;

La granulation humide du mélange est obtenue par l'introduction d'une quantité suffisante d'eau sous agitation vigoureuse. Si le liant n'a pas été ajouté lors de l'étape de mélange à sec, il est ajouté à l'eau et solubilisé avant granulation.
- séchage du granulé jusqu'à l'humidité résiduelle requise de toute manière connue, de préférence en lit d'air fluidisé ;
- ajout de l'éventuel complément de liant et du reste des excipients et mélange ;
- ajout du fumarate de stéaryle de sodium et mélange ;
- compression et récupération des comprimés.

La mise en œuvre de ce procédé peut être réalisée à l'aide de tout appareil approprié à cet effet, les mélangeurs granulateurs généralement utilisés ou les sécheurs granulateurs sont bien connus de l'homme de l'art. Les granulés sont séchés, calibrés et mélangés aux autres composants.

### Figures

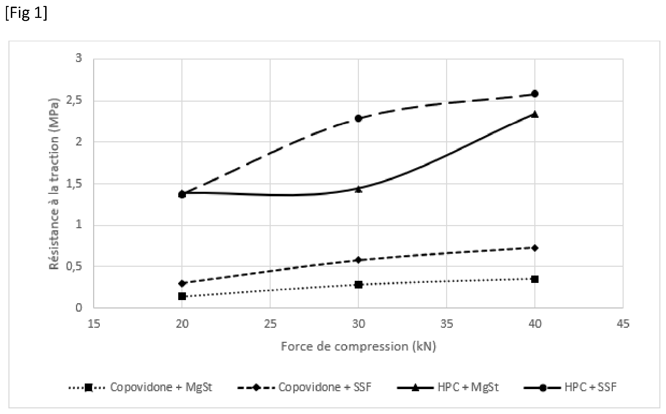

Figure 1 : graphe illustrant les résultats de tests comparatifs de résistance à la traction de comprimés préparés par compactage.

### EXEMPLES

### I. Fabrication des comprimés d'argile

### I.A. Préparation des comprimés par compactage (granulation sèche) et compression

Ce procédé comprend globalement deux principales étapes : le compactage et la compression :
- de façon générale, la granulation sèche est un procédé qui vise à former des grains à partir de poudre. La poudre est comprimée entre deux rouleaux pour former un ruban, qui est ensuite broyé à travers des grilles de pré-calibrage et de calibrage pour obtenir des grains. Le compactage à rouleaux est une technique de granulation sèche permettant l'agglomération des particules d'un mélange de poudre afin de lui conférer des propriétés nécessaire pour obtenir des comprimés répondant aux exigences industrielles et réglementaires- la compression est réalisée selon le principe suivant : une matrice, dans laquelle coulisse un poinçon inférieur, crée un volume (chambre de dosage) dans lequel on introduit un mélange de poudre comprenant le liant et le lubrifiant et d'argile compactée. Un poinçon supérieur vient fermer ce volume et, avec le poinçon inférieur, comprime le mélange jusqu'à obtenir un comprimé. Le poinçon supérieur se retire, le poinçon inférieur remonte et éjecte le comprimé. Plus précisément, les comprimés ont été fabriqués à l'aide d'un simulateur de compression STYL'One Evo avec un outillage permettant de préparer les comprimés selon la forme spécifique souhaitée, dans le cas présent, la forme choisie pour les essais réalisés est EU-B 19 X 10 mm R34.8/R4.6 - de forme ovale avec ligne de rupture.

Dans le cadre de la mise au point des comprimés selon l'invention, les formulations suivantes ont été préparées :

**[table 1]**

| | **I** | **II** | **III** | **IV** |
|---|---|---|---|---|
| Argile (smectite) | 89% | 89% | 89% | 89% |
| Copovidone | 10% | 10% | / | / |
| Hydroxypropylcellulose | / | / | 10% | 10% |
| stéarate de magnésium | 1% | / | 1% | / |
| Fumarate de stéaryle de sodium | / | 1% | / | 1% |

**[table 2]**

| | A | B | C |
|---|---|---|---|
| Argile (smectite) | 89% | 90,8% | 86% |
| Hydroxypropylcellulose | 10% | 8% | 8% |
| Amidon prégélatinisé | / | / | 5% |
| Fumarate de stéaryle de sodium | 1% | 1,2% | 1% |

### I.B. Préparation des comprimés par granulation humide et compression

Les essais de granulation humide à fort cisaillement (High Shear Wet Granulation, HSWG) ont été réalisés à l'aide du granulateur à fort cisaillement Mi-Pro (PROCEPT, BELGIQUE). Une buse bi-fluide (BF) avec un orifice de buse de 0,4 mm a été utilisée pour délivrer le liquide en utilisant un gaz d'atomisation de 3,0 L/min. Pour chaque essai, de l'eau a été utilisée comme liquide de granulation. La durée de l'essai a été fixée à 10 minutes avec un temps de mouillage de 2 minutes.

L'étape de séchage a ensuite été réalisée à l'aide du lit d'air fluidisé 4M8Trix à l'échelle laboratoire (PROCEPT, Belgique) avec une plaque de fond maillée.

Enfin, les comprimés ont été fabriqués à l'aide d'un simulateur de compactage STYL'One Evo avec un outillage permettant de préparer les comprimés selon la forme spécifique souhaitée, dans le cas présent, la forme choisie pour les essais réalisés est EU-B 19 X 10 mm R34.8/R4.6 - de forme ovale avec ligne de rupture.

**[table 3]**

| | D | E | F | G |
|---|---|---|---|---|
| Argile (smectite) (phase interne) | 86,8% | 85,0 % | 83,2 % | 86,8% |
| Hydroxypropylcellulose (phase interne) | 2,68% | 2,68% | 2,68% | 2,69 % |
| Hydroxypropylcellulose (phase externe) | / | / | / | 10% |
| Glycolate d'amidon sodique (phase interne) | / | 1,79% | 3,58 | / |
| L-HPC (Phase externe) | 10% | 10% | 10% | / |
| fumarate de stéaryle de sodium (phase externe) | 0,5% | 0,5% | 0,5% | 0,5% |

### Il. Evaluation des caractéristiques des comprimés

### II.A. Méthodes analytiques

### II.A.1. Dosage de la smectite

### Principe :

Le dosage de la smectite est réalisé par gravimétrie après calcination de l'ensemble des excipients.

La perte à la calcination est la perte de masse obtenue à 900°C ± 25°C.

La teneur en smectite est calculée en tenant compte de la perte de masse à la calcination de la matière première entrée en fabrication.

### Mode opératoire :

Peser un comprimé à l'aide d'un mortier pilon, le broyez.

Dans un creuset préalablement taré (T), introduire le broyat du comprimé et le peser (Pe en g).

Introduire le creuset dans le four. Réaliser une montée de température de température ambiante à 900°C en 2 heures suivi d'un plateau à 900°C pendant 1 heure.

Laisser refroidir le creuset contenant le résidu, puis peser le creuset avec le résidu (Tr).

### Calcul :

La teneur en smectite exprimée en g par comprimé, est donnée par l'expression : [Math 1] $\frac{\left(Tr\right) - \left(T\right)}{\left(T+Pe\right) - \left(T\right)} \times \frac{100}{100 - A} \times {M}_{cp}$ Où
- Tr :: Masse de la tare et du résidu, en g
- T :: Masse de la tare, en g
- T+Pe :: Masse de la Tare et de la prise d'essai, en g
- A :: Perte à la calcination de la matière première entrée en fabrication en g/100g
- Mcp :: Masse réelle du comprimé sur lequel est réalisé le dosage, en g

### II.A.2. Mesure du pouvoir adsorbant avec l'hexacobalt (trichlorure de cobaltihexammine)

### Principe

La méthode de détermination du pouvoir adsorbant, aussi appelée capacité d'échange cationique (CEC) et des cations échangeables (AI, Ca, Fe, K, Mg, Mn, Na) à l'aide d'une solution de trichlorure de cobaltihexammine, est une méthode de dosage quantitatif du trichlorure de cobaltihexammine restant en solution (non adsorbée par l'échantillon) après mise en contact avec l'argile.

La quantité de trichlorure de cobaltihexammine non adsorbée par l'échantillon d'argile est déterminée par spectrophotométrie d'absorption dans le visible à une longueur d'onde de 475 nm.

La mise en œuvre de cette méthode est effectuée selon la norme AFNOR 23470 NF ISO.

### Matériel

- Chlorure de cobaltihexammine (III), pureté ≥ 99%, Numéro CAS: 10534-89-1, référence VWR A15470 ou équivalent,
- Papier filtre quantitatif sans cendre Sodipro grade 14 diamètre 125 mm à filtration lente, référence Sodipro 6050041-06 ou équivalent.
- Cuve en quartz de 10 mm

### Mode opératoire : préparation des solutions

Préparation du témoin de la solution de trichlorure de cobaltihexammine : 20 mL de trichlorure de cobaltihexammine 0,0166 mol/L.

Préparation de la solution échantillon : un demi comprimé et 50,0 mL de trichlorure de cobaltihexammine à 0,0166 mol/L.

Préparation de la solution de référence : 0,70 g d'échantillon en poudre et 50,0 mL de trichlorure de cobaltihexammine à 0,0166 mol/L

### Mesure

Mesurer l'absorbance des solutions (eau purifiée, témoin, smectite témoin et échantillons) à 475 nm.

### Calcul du pouvoir adsorbant

*Pouvoir adsorbant des échantillons :* La quantité de trichlorure de cobaltihexammine adsorbée, exprimée en milliéquivalents/100 grammes de smectite, est donnée par l'expression suivante : [Math 2] $Adsorption \left(mEq/100g\right) = \frac{\left(A1-A2\right) \times C \times 50 \times Mcp \times 3}{A 1 \times Pe \times D} \times 100$ Où,
- C: Concentration de la solution de trichlorure de cobaltihexammine, 0,0166 mmol/mL
- A1: Absorbance de la solution de référence de trichlorure de cobaltihexammine
- A2: Absorbance de l'échantillon
- Pe: Prise d'essai (g)
- M_{cp}: Masse théorique d'un comprimé en grammes dans le cas d'une prise d'essai de poudre Masse réelle du comprimé en grammes dans le cas d'une prise d'essai en comprimé
- D: Teneur en smectite du lot en grammes
- 3: Nombre de cations échangés

*Pouvoir adsorbant de la smectite de référence :* La quantité de trichlorure de cobaltihexammine adsorbée, exprimée en milliéquivalents/100 grammes de smectite de référence, est donnée par l'expression suivante : [Math 3] $Adsorption \left(mEq/100g\right) = \frac{\left(A1-A2\right) \times C \times 50 \times 3}{A 1 \times Pe} \times \frac{100}{100 - Hr} \times 100$ Où,
- Hr: Perte à la dessiccation de la Smectite de référence

### II.A.3. Analyse de la taille des particules d'argile par diffraction de la lumière laser

*Équipement :* Analyseur de taille de particules de type Malvern Mastersizer 3000 ou équivalent, équipé en voie humide

### Préparation des échantillons :

Disperser une dose de produit, soit 2 comprimés dans 100 ml d'eau Milli Q

Disperser pendant 1 heure sous agitation magnétique

### Analyse :

Les suspensions obtenues sont analysées selon les recommandations du fabricant.

### II.A.4. Mesure du temps de désagrégation

Le test de désagrégation évalue si les comprimés se désagrègent dans le temps prescrit par la pharmacopée en mimant le péristaltisme humain dans une eau thermostatée à 37°C. Ce test permet de s'assurer de la dispersion rapide de l'actif dans les fluides du tractus gastrointestinal et de garantir l'efficacité de la forme.

Il est effectué par agitation standardisée du comprimé, dans le milieu liquide (l'eau en général) à 37° C, dans un tube dont le fond est grillagé. Le test est terminé quand il n'y a plus de résidus de comprimés sur la grille du panier.

Une durée limite maximale de désagrégation est fixée pour chaque type de comprimé. Dans le cas des comprimés à libération immédiate, le temps de désagrégation doit être inférieure à 1800 secondes.

### II.A.5. Mesure de la résistance à la traction

La courbe « Pression - résistance à la traction » permet la caractérisation de la dureté relative d'un produit. Cette courbe est indépendante des propriétés de l'outillage utilisé (forme du poinçon, épaisseur finale du comprimé, etc.).

La pression moyenne de compression correspond à l'effort appliqué sur le poinçon en fonction de la surface projetée des poinçons :
Pr : Pression moyenne de compression (MPa)
F : Force de compression moyenne (kN) [Math 4] $Pr = \frac{F}{PS} ∗ 1000$
PS : Surface projetée des poinçons (mm2)
Le coefficient 1000 correspond à a conversion d'unités.

### II.A.6. Evaluation de la friabilité

Le test de friabilité permet de s'assurer que les comprimés présentent une résistance mécanique suffisante, pour que leurs surfaces ne soient pas endommagées ou ne présentent pas des signes d'abrasion ou de rupture, sous l'effet de toutes les manipulations (chocs mécaniques, frottements, attrition).

La perte en masse d'un comprimé est estimée par application d'un stress sur celui-ci. Ce stress est généré par un tambour qui effectue des rotations (tour/min) durant un temps donné (25tr/min pendant 4min selon la Pharmacopée Européenne 2.9.7).

Pour cela, 6,5 g de comprimés sont pesés avant et après le stress. La perte de masse maximale acceptable dans l'industrie pharmaceutique est de 1% de la masse des comprimés soumis à l'essai.

### II.B. Résultats

### II.B.1. Tests comparatifs de résistance à la traction de comprimés préparés par compactage

Les comprimés testés correspondent aux formulations détaillées dans la partie I.A.

Pour présenter des propriétés satisfaisantes, les comprimés doivent avoir une résistance à la traction d'au moins 1,0 MPa et de préférence d'au moins 1,5 MPa.

Les résultats obtenus sont présentés dans le tableau 4 et représentés graphiquement à la Figure 1 :

**[table 4]**

| **Force de compression (kN)** | **Resistance à la traction (MPa)** | | | |
|---|---|---|---|---|
| | I | II | III | IV |
| | Copovidone + MgSt | Copovidone + SSF | HPC + MgSt | HPC + SSF |
| **20** | 0,141 | 0,301 | 1,383 | 1,375 |
| **30** | 0,287 | 0,577 | 1,441 | 2,289 |
| **40** | 0,356 | 0,729 | 2,341 | 2,582 |

### Légende : MgSt = magnésium de stéarate, SSF = fumarate de stéaryle de sodium, HPC = hydroxypropylcellulose.

Ces résultats montrent que la sélection de l'hydroxypropylcellulose en tant que liant et le fumarate de stéaryle de sodium comme lubrifiant améliore de façon inattendue et très significative la résistance à la traction des comprimés.

### II.B.3. Temps de désagrégation

Le temps de désagrégation doit être inférieur ou égale à 30 minutes (soit 1800 secondes). Les résultats obtenus sont présentés dans le tableau 5 ci-dessous.

### II.B.4. Dosage en smectite

Le dosage en smectite est considéré comme conforme lorsque les comprimés comprennent entre 1,43 et 1,57 g de smectite par comprimé.

Les résultats obtenus sont présentés dans le tableau 5 ci-dessous.

### II.B.5. Pouvoir absorbant

Le pouvoir absorbant est considérée comme conforme lorsque les comprimés sont capables d'absorber entre 80 to 150 mEq d'hexacolbalt/100 g d'argile

Les résultats obtenus sont présentés dans le tableau 5 ci-dessous.

### II.B.6. Granulométrie après dispersion dans l'eau

La granulométrie des particules d'argile après dispersion dans l'eau doit être telle que la valeur du D90 est inférieure ou égale à 200 µm.

Les résultats obtenus sont présentés dans le tableau 5 ci-dessous.

**[table 5]**

| **Formule** | **Dosage smectite (g/cp)** | **Pouvoir absorbant (hexacobalt)** | **Friabilité (%) <1%** | **Granulométrie D90 après dispersion voie humide** | **Temps de désagrégation < 1800 s** |
|---|---|---|---|---|---|
| A | 1,48 | 100,7 | <1 | 39,9 µm | < 508 |
| B | 1,48 | 101,1 | <1 | 36,9 µm | < 643 |
| C | 1,51 | 100,8 | <1 | 32,3 µm | < 358 |
| D | 1,49 | 106,3 | <1 | 77,5 µm | < 120 |
| E | 1,47 | 101,8 | <1 | 114 µm | < 300 |
| F | 1,53 | 108,1 | <1 | 154 µm | < 330 |
| G | 1,46 | 95,2 | <1 | 45,9 µm | <1440 |

### Conclusion :

Ces résultats montrent que les propriétés des comprimés de smectite selon l'invention répondent parfaitement aux exigences imposées.

## Revendications

1. Comprimé d'argile consistant en :
- au moins 80% en poids d'argile ;
- de 0,5 à 1,5% en poids de fumarate de stéaryle de sodium ;
- de 5 à 15% en poids, de préférence, entre 8 et 10% en poids, d'hydroxypropylcellulose à titre de liant ;
- optionnellement, de 5 à 15% en poids d'un diluant choisi parmi la cellulose microcristalline, l'amidon et l'amidon prégélatinisé, la maltodextrine, le mannitol, le phosphate dicalcique ; et
- optionnellement, de 2 à 10% en poids d'un désintégrant choisi parmi l'hydroxypropylcellulose faiblement substitué (L-HPC), le croscarmellose sodium, le glycolate d'amidon sodique, la crospovidone, les polysaccharides de soja, l'amidon et l'amidon prégélatinisé.

2. Comprimé d'argile selon la revendication 1, **caractérisé en ce que** l'argile est choisie parmi la smectite et l'attapulgite, de préférence, l'argile est une smectite.

3. Comprimé d'argile selon la revendication 1 ou la revendication 2, **caractérisé en ce qu'**il comprend au moins 83% en poids d'argile, préférentiellement, entre 85 et 95% en poids.

4. Comprimé d'argile selon l'une quelconque des revendications précédentes, **caractérisé en ce qu'**il comprend entre 0,8 et 1,2% en poids de fumarate de stéaryle de sodium.

5. Comprimé d'argile selon l'une quelconque des revendications précédentes, **caractérisé en ce qu'**il ne comprend pas de désintégrant.

6. Comprimé d'argile selon l'une quelconque des revendications précédentes, **caractérisé en ce qu'**il ne comprend pas de diluant.

7. Comprimé d'argile selon l'une quelconque des revendications précédentes, **caractérisé en ce qu'**il a un poids compris entre 0,5 et 2,5 g, de préférence, entre 1 et 2 g.

8. Comprimé d'argile selon l'une quelconque des revendications précédentes, **caractérisé en ce qu'**il a :
- un temps de désagrégation inférieur ou égal à 30 minutes (1800 secondes), de préférence inférieur ou égal à 15 minutes (900 secondes) ; et/ou
- une dose d'argile dans le comprimé fini comprise entre 95 et 105% de la teneur cible ; et/ou
- un pouvoir absorbant tel que l'absorption d'hémisulfate de strychnine est comprise entre 307 et 575 mg d'hémisulfate de strychnine par g d'argile ou l'absorption d'hexacolbalt est comprise entre 80 et 150 mEq d'hexacolbalt par 100 g d'argile ; et/ou
- une distribution granulométrique des particules après dispersion telle que la D₉₀ est inférieure à 200 µm ; et/ou
- une stabilité mécanique telle que la perte en poids des comprimés est inférieure à 1% lors de l'évaluation par le test de friabilité selon la Pharmacopée Européenne 2.9.7..

9. Comprimé d'argile selon l'une quelconque des revendications précédentes pour son utilisation pour le traitement de désordres gastro-intestinaux tels que troubles fonctionnels du tractus gastro-intestinal comme la diarrhée, éventuellement chronique ou aigue.

10. Procédé de préparation du comprimé selon l'une quelconque des revendications 1 à 8, comprenant les étapes suivantes :
- compactage de l'argile seule ou avec un ou plusieurs excipients ;
- mélange de l'argile compactée et des excipients
- compression et récupération des comprimés.

11. Procédé de préparation du comprimé selon l'une quelconque des revendications 1 à 8, comprenant les étapes suivantes :
- mélange à sec de l'argile seul ou avec tout ou partie du liant et un ou plusieurs excipients à l'exception du fumarate de stéaryle de sodium ;
- granulation humide avec une partie du liant s'il n'a pas été ajouté lors de l'étape de mélange à sec ;
- séchage du granulé jusqu'à l'humidité résiduelle requise de toute manière connue, de préférence en lit d'air fluidisé ;
- ajout de l'éventuel complément de liant et mélange ;
- ajout du fumarate de stéaryle de sodium et mélange ;
- compression et récupération des comprimés.
